# EUROPÄISCHE PATENTSCHRIFT

(11) **EP 1 762 196 B1**
(45) Veröffentlichungstag und Bekanntmachung des Hinweises auf die Patenterteilung: **12.11.2008**
(21) Anmeldenummer: 06018029.6
(22) Anmeldetag: 29.08.2006
(51) Int. Cl.: A61B 17/70

(54) **Dynamische Klemmvorrichtung für Wirbelsäulenimplantat**
Dynamic clamping device for spinal implant
Dispositif de serrage dynamique pour implant rachidien

(30) Priorität: 13.09.2005 CH 14882005; 21.11.2005 US 738759 P
(43) Veröffentlichungstag der Anmeldung: 14.03.2007
(73) Patentinhaber: BIRD Biedermann AG, 6072 Sachseln (CH)
(72) Erfinder: Freudiger, Stefan, 3047 Bremgarten (CH)
(74) Vertreter: Hofer, Dorothea

(56) Entgegenhaltungen:
- US-A1- 2004 138 660

## Beschreibung

Die vorliegende Erfindung betrifft ein dynamisches Stabilisiersystem für Wirbelsäulen, welches die Wirbelsäule ohne Versteifung zu stabilisieren vermag. Die elastischen Verbindungselemente werden hierbei gemäss Patentanspruch 1 auf eine neuartige Weise mit den Knochen- respektive Pedikelschrauben verbunden.

Gemäss des Standes der Technik gibt es für metallische Stäbe eine Vielzahl von Stab-/Schraubenverbindungen, welche vor allem für Fusionsoperationen (Versteifungen) zur Anwendung gelangen. Elastische Systeme, welche die Wirbelsäulensegmente lediglich stützen und stabilisieren, nicht aber versteifen, sind erst vereinzelt erschienen und dementsprechend sind auch Vorrichtungen für den Zusammenschluss der elastischen Verbindungselemente mit den Pedikelschrauben erst vereinzelt vorhanden.

Grundsätzlich sind Stab-/Schraubenverbindungen, wie sie für metallische Stäbe geeignet sind, nicht automatisch auch für elastische Verbindungselemente geeignet, da zum Beispiel elastische Stäbe aus Kunststoff anderen Gesetzmässigkeiten unterliegen als vergleichsweise steife Stäbe aus Metall. So können elastische Kunststoff-Stäbe nicht einfach dauerhaft mit Kraftschluss geklemmt werden, da sie in der Regel die Klemmkraft durch Fliessen wieder vermindern können. Es sind also Verbindungskonzepte gesucht, welche nebst einer allfälligen kraftschlüssigen (Basis-) Klemmung auch einen Anteil Formschluss aufweisen oder idealerweise einen dynamischen Formschluss, welcher sich mit zunehmender Belastung vorübergehend verstärkt und sich nach der Belastung wieder vermindert.

Es werden im Folgenden bekannte Stab-/Schraubenverbindungen aufgeführt und ihre Nachteile in Bezug auf die vorliegende Erfindung erläutert.

Die nachstehenden Erfindungen weisen zwar ebenfalls drehbare Halteelemente auf, welche jedoch einzig und alleine dazu dienen, auch nicht senkrecht zu der Schraubenachse verlaufende Stäbe mit der Schraube zu verbinden. Diese Haltelemente sind also nur während der Befestigungsphase drehbar und sind nach Abschluss derselben nicht mehr beweglich: Patentanmeldung WO 01/06939 (Martin Benlloch et al.); Patentanmeldung WO 97/43974 (Dombrowski et al.); Patentschrift US 4,987,892 (Krag und Pope).

US 2004/0138660 A1 beschreibt ein Wirbelsäulenimplantat bestehend aus einer Polyaxialschraube und einem Verschlusskappenaufbau für diese. Der Verschlusskappenaufbau hat ein äußeres Verschlusselement und ein Eingriffselement, welches einen dem Stab zugewandten deformierbaren Ring aufweist. Der Stab ist aus einem starren Material, wie beispielsweise aus Titan oder einer Titanlegierung gebildet und der Ring deformiert sich, wenn die Verschlusskappe aufgeschraubt wird. So erhält der Bediener durch die Deformierung des Rings eine Rückmeldung, die es ihm erlaubt, zu bestimmen, ob die Verschlusskappe schon fest genug aufgeschraubt ist.

Der vorliegenden Erfindung liegen demnach die Aufgaben zugrunde, einen elastischen Kunststoff-Stab mit kontinuierlicher glatter Oberfläche stufenlos und sicher mit einer Knochen-, respektive Pedikelschraube zu verbinden und dabei sowohl Zug- und Druck- wie auch Scher- und Torsionskräfte zwischen benachbarten Wirbelkörpern zu übertragen.

Die Lösung dieser Aufgabe zeichnet sich dadurch aus, dass die Verbindung eine Kombination aus dauerhaftem (Basis-) Kraftschluss und dynamischem Formschlussanteil darstellt. Der dynamische Formschlussanteil wird dabei durch lokale elastische, respektive plastische Deformation des Kunststoff-Stabes erzielt. Dieser dynamische Formschlussanteil verstärkt sich automatisch mit zunehmender Achsialkraft im Stab und vermindert sich anschliessend wieder automatisch mit abnehmender Achsialkraft im Stab.

Gegenstand der Erfindung ist demzufolge die im Patentanspruch 1 definierte kraftschlüssige Schrauben-/Stabverbindung mit dynamischem Formschlussanteil. Diese ist in der Lage, einen glatten elastischen Kunststoff-Stab derart mit dem Kopf einer Knochen-, respektive Pedikelschraube zu verbinden, dass die zu erwartenden Kräfte, aufgrund der Anwendung als dynamische Stabilisierung der Lendenwirbelsäule, dauerhaft und sicher übertragen werden können. Dabei liegt der Vorteil in der Kombination einer einfachen und stufenlos positionierbaren kraftschlüssigen Verbindung mit der Zuverlässigkeit eines formschlüssigen Verbindungsanteils. Der initiale Formschluss wird dabei durch eine dynamische Zunahme des Formschlusses mit zunehmender Achsialkraft unterstützt, welche nach Abnahme der Achsialkraft ebenfalls wieder abnimmt. Damit wird der Kunststoff-Stab geschont und ein Fliessen unter Last wird weitgehend verhindert.

Im Folgenden wird die vorliegende Erfindung anhand der beiliegenden Zeichnungen, welche lediglich Ausführungsbeispiele darstellen, näher erläutert.

Es zeigen schematisch:
Fig. 1a ein elastisches Verbindungselement (2) in einer (geschnittenen) Pedikelschraube (1) mit einem Füllstück (3) in Neutralstellung um einen Drehpunkt (4) und Fig. 1b ein elastisches Verbindungselement (2) unter Zugkraft mit einem Füllstück (3) in rotierter Stellung um einen Drehpunkt (4).
Fig. 2a eine Seitenansicht eines beispielsweise runden Verbindungselementes (2) in einer Pedikelschraube (1) mit einem Füllstück (3), einem Drehpunkt mit Kugeloberfläche (4a) und einem Klemmelement (5); Fig. 2b eine Frontal- und Fig. 2c eine Draufsicht derselben Elemente.
Fig. 3a eine Seitenansicht eines beispielsweise runden Verbindungselementes (2) in einer Pedikelschraube (1) mit einem Füllstück (3), einem Drehpunkt in einer Achse (4b) und einem Klemmelement (5); Fig. 3b eine Frontal- und Fig. 3c eine Draufsicht derselben Elemente.
Fig. 4a ein elastisches Verbindungselement (2) in einer (geschnittenen) Pedikelschraube (1) mit dem Füllstück (3) in Neutralstellung um den Drehpunkt (4) und Fig. 4b ein elastisches Verbindungselement (2) unter Zugkraft mit dem Füllstück (3) in rotierter Stellung um den Drehpunkt (4). Hierbei weist das Füllstück (3) Rippen (6) auf.
Fig. 5 eine Draufsicht eines beispielsweise runden Verbindungselementes (2) in einer Pedikelschraube (1) mit Schraubenflügeln (1a), mit einem Füllstück (3), einem Drehpunkt mit Kugeloberfläche (4a) und einem Klemmelement (5), welches mit der Schraube über ein Sägezahngewinde (7) verbunden ist.
Fig. 6 eine Draufsicht eines beispielsweise runden Verbindungselementes (2) in einer Pedikelschraube (1), mit einem Füllstück (3), einem Drehpunkt in einer Achse (4b) und derselben Achse als Klemmelement (5).

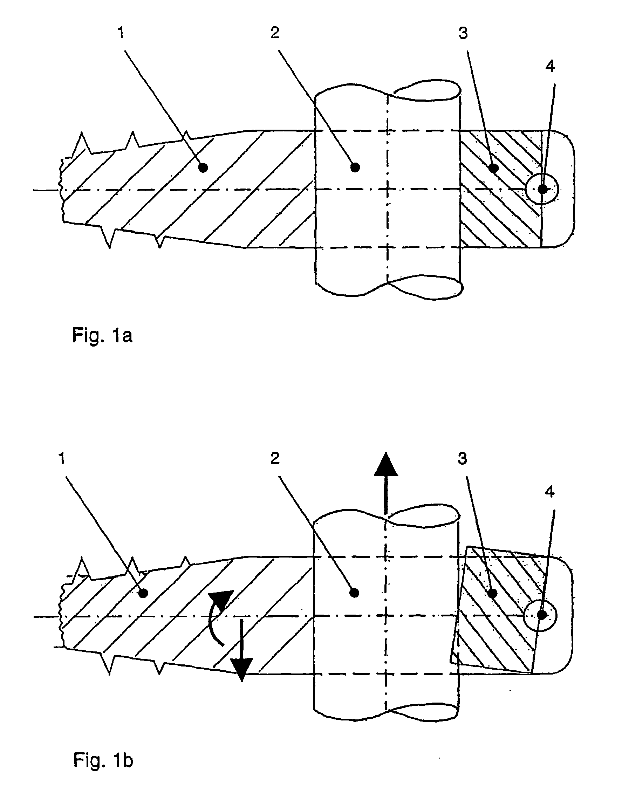

Fig. 1 a stellt ein elastisches Verbindungselement (2) in einer (geschnittenen) Pedikelschraube (1) mit einem drehbaren Füllstück (3) in Neutralstellung um einen Drehpunkt (4) dar. Fig. 1b zeigt wie sich das Füllstück (3) dreht, sobald das elastische Verbindungselement (2) unter Zug, rsp. Dehnung gesetzt wird. Hierbei ist das Füllstück (3) so angeordnet, dass seine der Kraftrichtung entgegengesetzte Kante in die Oberfläche des elastischen Verbindungselementes eindringt und einen Formschlussanteil erzeugt, welcher die Verankerungskraft deutlich erhöht.

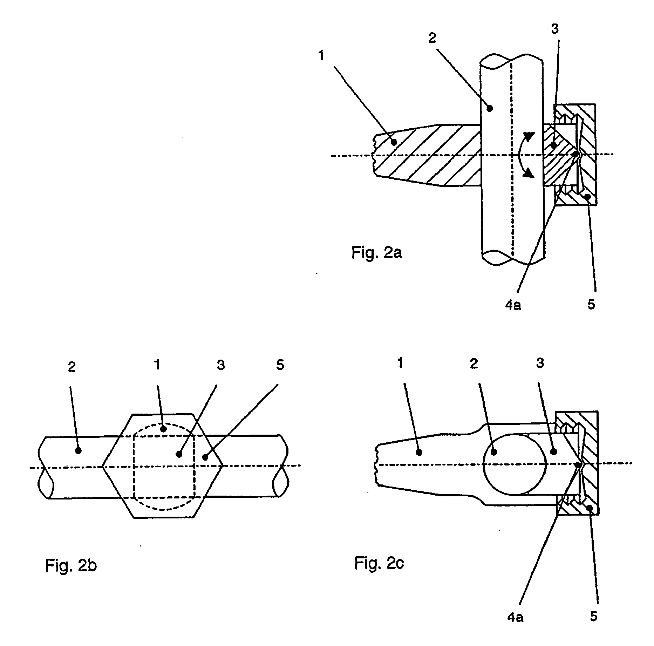

Fig. 2a, 2b und 2c stellen eine Seitenan-, Frontal- und Draufsicht des elastischen Verbindungselementes (2), der Pedikelschraube (1), des Füllstückes (3) und des Klemmelementes (5) dar. Hierbei wird das drehbare Füllstück (3) über eine Kugeloberfläche (4a) direkt vom Klemmelement (5) gegen das Verbindungselement (2) gedrückt.

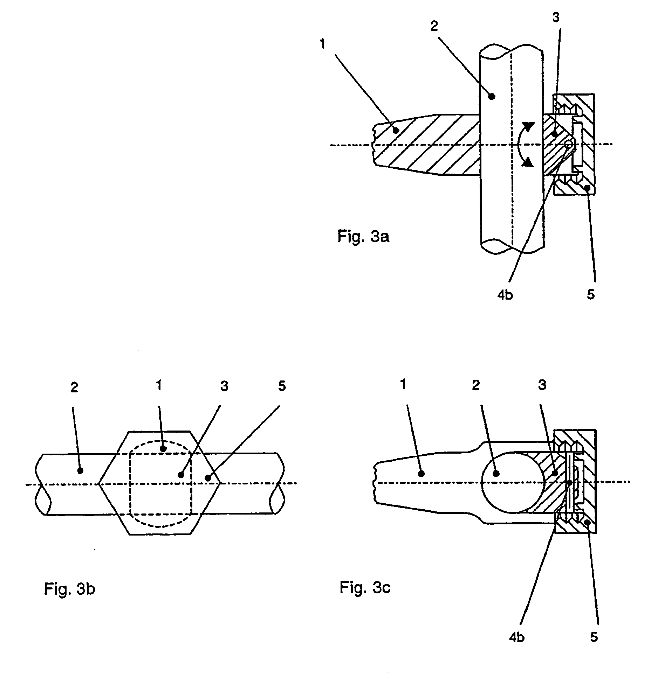

Fig. 3a, 3b und 3c stellen eine Seitenan-, Frontal- und Draufsicht des elastischen Verbindungselementes (2), der Pedikelschraube (1), des Füllstückes (3) und des Klemmelementes (5) dar. Hierbei wird das drehbare Füllstück (3) über eine Achse (4b) vom Klemmelement (5) gegen das Verbindungselement (2) gedrückt.

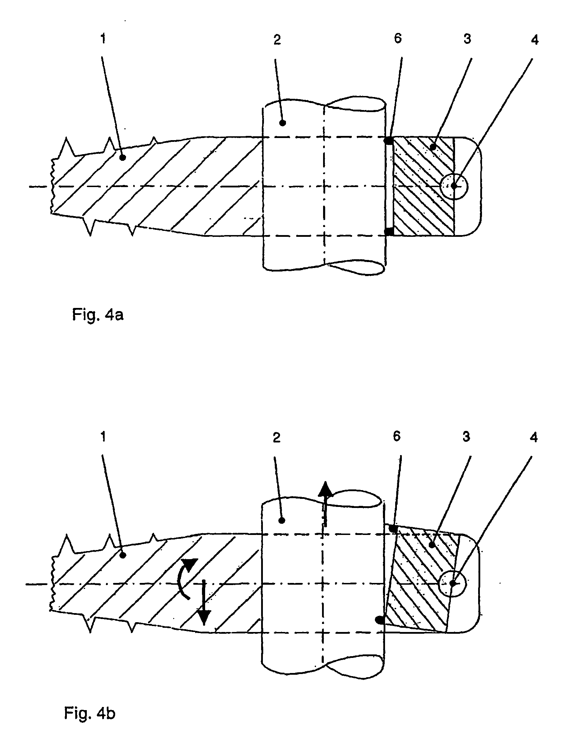

Fig. 4a stellt wiederum ein elastisches Verbindungselement (2) in einer (geschnittenen) Pedikelschraube (1) mit einem drehbaren Füllstück (3) in Neutralstellung um einen Drehpunkt (4) dar. Fig. 4b zeigt wie sich das Füllstück (3) dreht, sobald das elastische Verbindungselement (2) unter Zug, rsp. Dehnung gesetzt wird. Zum besseren Eindringen in die Oberfläche des elastischen Verbindungselementes weist das Füllstück (3) Rippen (6) auf. Diese Rippen (6) dringen auch in Neutralstellung leicht in die Oberfläche des elastischen Verbindungselementes (2) ein und erhöhen somit den Anfangswiderstand am Füllstück (3), was die Drehung desselben in Kraftrichtung unterstützt.

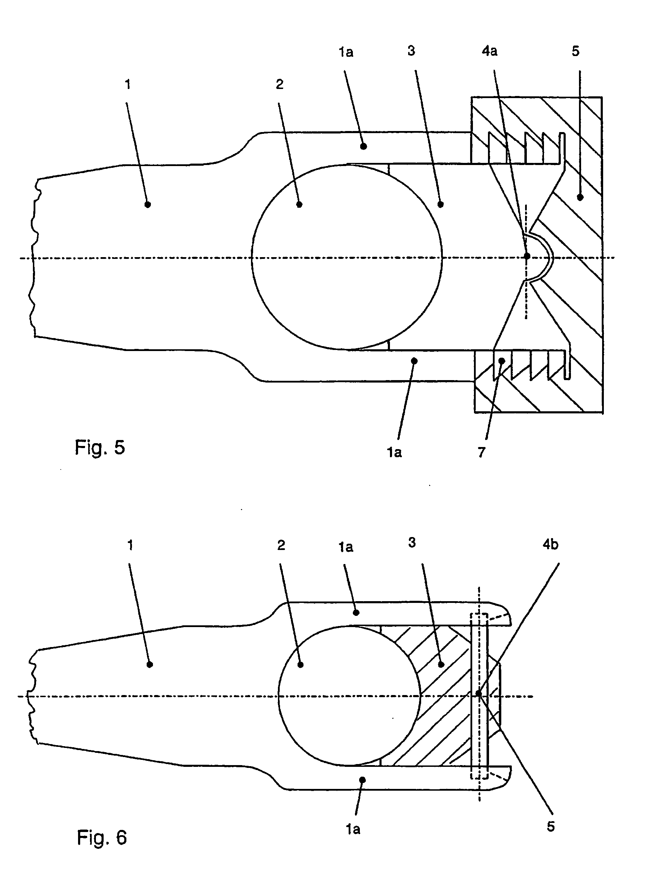

Fig. 5 zeigt eine Draufsicht eines beispielsweise runden Verbindungselementes (2) in einer Pedikelschraube (1) mit einem Füllstück (3), einem Drehpunkt mit Kugeloberfläche (4a) und einem Klemmelement (5), welches mit der Schraube über ein Sägezahngewinde (7) verbunden ist. Hierbei vermeidet das Sägezahngewinde ein Zusammendrücken der beiden Schraubenflügel (1a) und damit eine allfällige Behinderung der Beweglichkeit des Füllstückes (3).

Fig. 6 zeigt eine Draufsicht eines beispielsweise runden Verbindungselementes (2) in einer Pedikelschraube (1), mit einem Füllstück (3), einem Drehpunkt in einer Achse (4b), wobei diese Achse gleichzeitig als Klemmelement (5) dient und durch elastisches Spreizen der Schraubenflügel (1a) in Position gebracht wird.

## Patentansprüche

1. Wirbelsäulenimplantat, bestehend aus einem elastischen Verbindungselement (2) mit einer Längsachse, mehreren Knochenschrauben (1), je einem Füllstück (3) und je einem Klemmelement (5) derart, dass das Verbindungselement (2) zwischen der Aufnahme im Schraubenkopf und dem Füllstück (3) durch das Klemmelement (5) kraftschlüssig geklemmt wird, wobei das Füllstück (3) drehbar ist und so angeordnet ist, dass seine der Kraftrichtung entgegengesetzte Kante in die Oberfläche des elastischen Verbindungselements eindringt, wodurch die Klemmung des Verbindungselementes unter zunehmender Achsialkraft in Richtung der Längsachse verstärkt wird.

2. Wirbelsäulenimplantat nach Anspruch 1, **dadurch gekennzeichnet, dass** sich das Füllstück (3) gegenüber dem Klemmelement (5) um einen Punkt mit einer Kugeloberfläche (4a) drehen kann.

3. Wirbelsäulenimplantat nach Anspruch 1, **dadurch gekennzeichnet, dass** sich das Füllstück (3) um eine Achse (4b) drehen kann, die vom Klemmelement (5) gehalten wird.

4. Wirbelsäulenimplantat nach einem der Ansprüche 1 bis 3, **dadurch gekennzeichnet, dass** das Füllstück (3) Rippen (6) aufweist zum wirksameren Eindringen in die Oberfläche des Verbindungselementes (2).

5. Wirbelsäulenimplantat nach Anspruch 4, **dadurch gekennzeichnet, dass** das Verbindungselement (2) ein Stab ist.

6. Wirbelsäulenimplantat nach Anspruch 5, **dadurch gekennzeichnet, dass** der Stab rund ist.

7. Wirbelsäulenimplantat nach einem der Ansprüche 1 bis 6, **dadurch gekennzeichnet, dass** das Verbindungselement (2) aus einem elastischen Kunststoff gefertigt ist.

8. Wirbelsäulenimplantat nach Anspruch 7, **dadurch gekennzeichnet, dass** das Verbindungselement (2) aus PCU (polycarbonateurethane) gefertigt ist.

9. Wirbelsäulenimplantat nach einem der Ansprüche 1 bis 8, **dadurch gekennzeichnet, dass** das Klemmelement (5) eine Mutter ist.

10. Wirbelsäulenimplantat nach Anspruch 9, **dadurch gekennzeichnet, dass** das Klemmelement (5) mit der Schraube (1) durch ein 5 Sägezahngewinde (7) verbunden ist.

11. Wirbelsäulenimplantat nach einem der Ansprüche 3 bis 8, **dadurch gekennzeichnet, dass** die Achse (4b) gleichzeitig als Klemmelement (5) dient.

## Claims

1. Spinal implant, comprising an elastic connection element (2) with a longitudinal axis, a plurality of bone screws (1), each with a filling piece (3) and with a clamping element (5), such that the connection element (2) is clamped with a force fit between the seat in the screw head and the filling piece (3) by means of the clamping element (5), the filling piece (3) being rotatable and being arranged in such a way that its edge counter to the direction of force penetrates into the surface of the elastic connection element, as a result of which the clamping of the connection element is strengthened with increasing axial force in the direction of the longitudinal axis.

2. Spinal implant according to Claim 1, **characterized in that** the filling piece (3) can rotate relative to the clamping element (5) about a point with a spherical surface (4a).

3. Spinal implant according to Claim 1, **characterized in that** the filling piece (3) can rotate about a pin (4b) held by the clamping element (5).

4. Spinal implant according to one of Claims 1 to 3, **characterized in that** the filling piece (3) has ribs (6) for more effective penetration into the surface of the connection element (2).

5. Spinal implant according to Claim 4, **characterized in that** the connection element (2) is a rod.

6. Spinal implant according to Claim 5, **characterized in that** the rod is round.

7. Spinal implant according to one of Claims 1 to 6, **characterized in that** the connection element (2) is made of a plastic material with elastic properties.

8. Spinal implant according to Claim 7, **characterized in that** the connection element (2) is made of PCU (polycarbonate urethane).

9. Spinal implant according to one of Claims 1 to 8, **characterized in that** the clamping element (5) is a nut.

10. Spinal implant according to Claim 9, **characterized in that** the clamping element (5) is connected to the screw (1) by a saw tooth thread (7).

11. Spinal implant according to one of Claims 3 to 8, **characterized in that** the pin (4b) simultaneously serves as clamping element (5).

## Revendications

1. Implant rachidien, composé d'un élément de liaison (2) élastique, ayant un axe longitudinal, d'une pluralité de vis à os (1), respectivement d'une pièce de remplissage (3) et respectivement d'un élément de serrage (5), de manière que l'élément de liaison (2) soit serré, avec une liaison à interaction de forces, entre le logement ménagé dans la tête de vis et la pièce de remplissage (3), au moyen de l'élément de serrage (5), la pièce de remplissage (3) étant susceptible de tourner et étant disposée de manière que son arête, opposée au sens d'action de la force, pénètre dans la surface de l'élément de liaison élastique, faisant que le serrage de l'élément de liaison est renforcé lorsque la force axiale, orientée dans la direction de l'axe longitudinal, augmente.

2. Implant rachidien selon la revendication 1, **caractérisé en ce que** la pièce de remplissage (3) peut tourner par rapport à l'élément de serrage (5), autour d'un point ayant une surface sphérique (4a).

3. Implant rachidien selon la revendication 1, **caractérisé en ce que** la pièce de remplissage (3) peut tourner autour d'un axe (4b), maintenu par l'élément de serrage (5).

4. Implant rachidien selon l'une des revendications 1 à 3, **caractérisé en ce que** la pièce de remplissage (3) présente des nervures (6), devant permettre une pénétration plus efficace dans la surface de l'élément de liaison (2).

5. Implant rachidien selon la revendication 4, **caractérisé en ce que** l'élément de liaison (2) est une barre.

6. Implant rachidien selon la revendication 5, **caractérisé en ce que** la barre est ronde.

7. Implant rachidien selon l'une des revendications 1 à 6, **caractérisé en ce que** l'élément de liaison (2) est fabriqué en une matière synthétique élastique.

8. Implant rachidien selon la revendication 7, **caractérisé en ce que** l'élément de liaison (2) est fabriqué en PCU (polycarbonate uréthane).

9. Implant rachidien selon l'une des revendications 1 à 8, **caractérisé en ce que** l'élément de serrage (5) est un écrou.

10. Implant rachidien selon la revendication 9, **caractérisé en ce que** l'élément de serrage (5) est relié à la vis (1) par un filetage en dents de scie (7).

11. Implant rachidien selon l'une des revendications 3 à 8, **caractérisé en ce que** l'axe (4b) sert simultanément d'élément de serrage (5).
